# FASCICULE DE BREVET EUROPEEN

(11) **EP 1 720 723 B1**
(45) Date de publication et mention de la délivrance du brevet: **13.02.2008**
(21) Numéro de dépôt: 05728111.5
(22) Date de dépôt: 22.02.2005
(51) Int. Cl.: B60H 3/00

(54) **DIFFUSEUR DE SUBSTANCES VOLATILES POUR VEHICULE AUTOMOBILE**
SPENDER FÜR FLÜCHTIGE SUBSTANZEN FÜR KRAFTFAHRZEUGE
VOLATILE SUBSTANCE DISPENSER FOR MOTOR VEHICLES

(30) Priorité: 26.02.2004 FR 0401942
(43) Date de publication de la demande: 15.11.2006
(73) Titulaire: Renault SAS, 92100 Boulogne Billancourt (FR)
(72) Inventeur: GUERRIER, Pierre, F-75020 Paris (FR); QUIRIN, Luc, F-75011 Paris (FR)
(86) Numéro de dépôt international: PCT/FR2005/050114
(87) Numéro de publication internationale: WO 2005/084974

(56) Documents cités:
- EP-A- 1 319 543
- WO-A-01/70284
- US-A- 4 537 351

## Description

L'invention concerne un diffuseur de substance(s) volatile(s) pour véhicule automobile, et plus particulièrement un diffuseur de type statique, comme divulgué par WO-01/702 84.

Les diffuseurs statiques présentent des plaquettes ou granules de substances, ou encore des matériaux imbibés de substances, dont les effluves sont dispersées lentement et irrégulièrement dans l'espace environnant. Afin d'améliorer la dispersion, certains diffuseurs sont accrochés aux ailettes des volets d'aération, par exemple au moyen d'un clip tel que décrit dans le document US-6 264 887, au moyen d'une fixation de type velcro tel que décrit dans le document US-4 813 344, ou encore au moyen d'un bloc élastomère compressible qui peut être inséré entre les ailettes du volet d'aération tel que décrit dans le document US-5 762 549. Certains diffuseurs sont introduits entièrement dans l'aérateur tel que dans le document US-5 269 723 ou présentent des moyens d'accrochage qui pénètrent entre les ailettes des volets d'aération tel que dans le document GB-2 263 404.

Ces différents diffuseurs nécessitent la réalisation de moyens d'accrochage relativement complexes. Ou encore, du fait de leur positionnement au milieu du flux d'air, ces dispositifs présentent l'inconvénient de se détacher lorsque le flux d'air est important, ou de se balancer ou de vibrer en produisant un bruit inconfortable pour les passagers.

L'invention vise à pallier ces inconvénients en proposant un diffuseur statique de substance(s) volatile(s) qui soit simple à réaliser et à mettre en place. Le diffuseur selon l'invention présente également l'avantage de pouvoir être placé à proximité immédiate d'un aérateur sans toutefois être situé au milieu du flux d'air, de manière à ne pas bouger avec le flux d'air.

A cet effet, l'objet de l'invention concerne un diffuseur de substance volatile destiné à un aérateur de véhicule automobile du type présentant une ouverture située au fond d'un renfoncement, caractérisé en ce qu'il présente un bord inférieur sensiblement de même forme et de mêmes dimensions que le pourtour de l'ouverture dudit aérateur et une paroi latérale en un matériau apte à être imprégné d'une ou plusieurs substances volatiles s'étendant depuis le bord en s'évasant, et en ce que le bord est pourvu de moyens de maintien aptes à maintenir le diffuseur sur le pourtour de l'ouverture de l'aérateur

Ainsi, une fois en place, le diffuseur n'est pas situé au milieu du flux d'air sortant de l'aérateur mais entoure ce dernier de sorte que le passage du flux d'air va permettre une dispersion de la ou des substances volatiles sans toutefois faire bouger le diffuseur.

Avantageusement, le diffuseur est pourvu de moyens d'étanchéité aptes à limiter le passage d'un flux d'air entre le bord du diffuseur et le pourtour de l'ouverture de l'aérateur. Ces moyens d'étanchéité permettent d'éviter le décollement du diffuseur sous l'effet de l'air sortant de l'aération.

Dans un mode de réalisation, les moyens de maintien et/ou d'étanchéité sont formés d'une protubérance s'étendant sur la face externe du bord sur toute la périphérie de celui-ci, les dimensions les plus grandes de la protubérance étant légèrement supérieures aux dimensions du pourtour de l'ouverture de l'aérateur. Un maintien efficace du diffuseur sur l'aérateur est ainsi obtenu de manière simple.

Dans une variante, la paroi latérale présente sensiblement la même forme que la paroi entourant l'ouverture de l'aérateur. Une telle forme en soi ou associée, par exemple, à un choix de couleurs coordonnées avec l'habitacle du véhicule, permet d'améliorer l'effet esthétique du diffuseur.

Avantageusement, le diffuseur est en matériau recyclable. Le diffuseur selon l'invention, destiné à être jeté lorsqu'il ne disperse plus de substance(s) volatile(s), peut être ainsi réalisé de manière très économique.

Avantageusement, le diffuseur est en matériau apte à être moulé. Un moule particulier sera alors utilisé pour chaque forme d'aérateur sur lequel on souhaite placer un diffuseur selon l'invention.

Avantageusement, le diffuseur est en papier ou carton.

Le diffuseur selon l'invention présente les avantages de pouvoir être réalisé avec un outillage, une matière première et une mise en oeuvre peu coûteux.

Par ailleurs, un tel diffuseur peut être imbibé de substances volatiles indifféremment pendant ou après sa fabrication. Par exemple, il peut être imbibé par l'utilisateur lui-même, qui peut alors choisir le produit qu'il va utiliser en fonction de ses goûts et besoins : parfum, produit stimulant les sens, produit d'assainissement de l'air, produit insecticide ou anti-acariens...

L'invention est maintenant décrite en référence aux dessins annexés, non limitatifs, dans lesquels :
- la figure 1 est une vue en perspective d'un diffuseur selon l'invention ;
- la figure 2 est une vue de dessus du diffuseur représenté figure 1;
- la figure 3 est une vue en coupe du diffuseur représenté sur les figures 1 et 2 placé sur un aérateur de véhicule automobile ;
- la figure 4 est un agrandissement de la partie encerclée de la figure 3 ;
- la figure 5 est une vue en coupe du diffuseur représenté sur les figures 1 et 2 placé sur un autre aérateur de véhicule automobile ;
- la figure 6 est un agrandissement de la partie encerclée de la figure 5.

Un mode de réalisation d'un diffuseur 1 selon l'invention est décrit en référence aux figures.

Le diffuseur 1 présente un bord inférieur 2 fermé, de forme circulaire, à partir duquel s'étend une paroi latérale 3 de forme évasée qui se termine par un bord supérieur 4, circulaire, de diamètre supérieur au diamètre du bord inférieur 2. La paroi latérale 3 s'étend sur tout le pourtour du bord inférieur 2.

Le diffuseur présente ainsi une forme générale d'entonnoir avec un orifice central. La face interne est définie comme étant la face délimitant cet orifice, par opposition à la face externe.

Dans l'exemple, le diffuseur présente une symétrie de révolution, les axes des deux bords circulaires étant confondus avec l'axe de révolution de la paroi latérale 3.

Le bord inférieur 2 présente sur toute sa périphérie une protubérance 5 qui s'étend sensiblement perpendiculairement à la paroi latérale 3 sur la face externe de celle-ci. Dans l'exemple, la protubérance 5 présente une forme annulaire dont le diamètre le plus grand est supérieur au diamètre le plus petit de la paroi latérale.

Le diffuseur 1 est réalisé en un matériau absorbant pouvant être imbibé d'une ou plusieurs substances volatiles (parfum ou autre). Il peut s'agir de papier, carton ou d'une matière polymère. De préférence, il est réalisé en carton recyclable, peu coûteux. Il peut s'agir par exemple d'un carton similaire à celui utilisé pour les boîtes à oeufs.

Le diffuseur 1 est conformé de manière à venir s'adapter sur un aérateur de véhicule automobile. A cet effet, le bord inférieur 2 présente sensiblement la même forme et les mêmes dimensions que l'ouverture d'un conduit d'aération. Le flux d'air issus de l'aérateur passe ainsi par l'orifice central de la forme d'entonnoir du diffuseur.

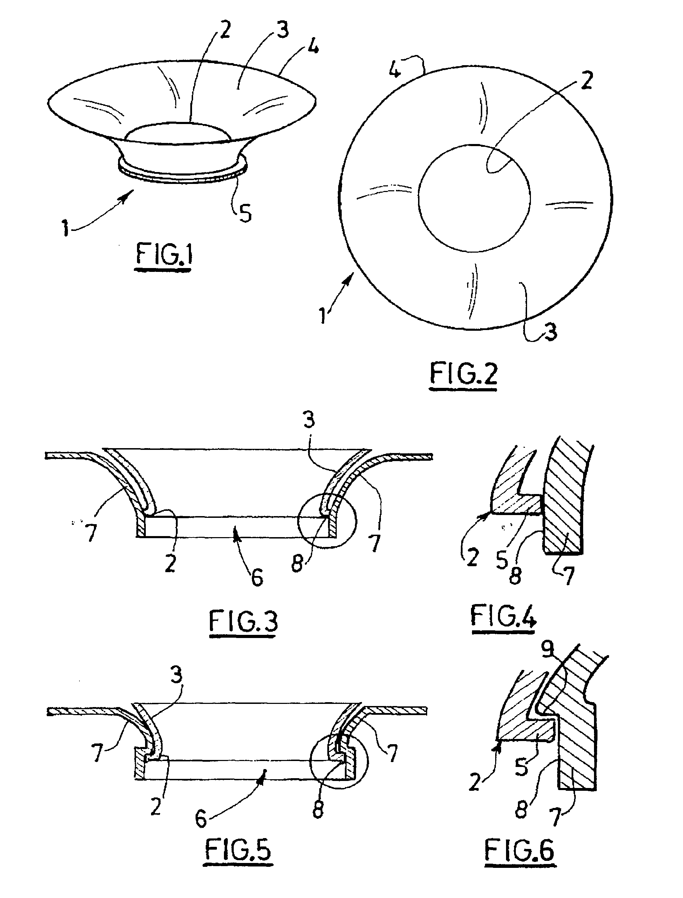

Les figures 3 et 5 représentent en coupe des ouvertures 6 d'un aérateur. Chaque ouverture 6 est généralement pourvue d'ailettes orientables non représentées. De telles ouvertures 6 sont ménagées dans les parois 7 d'une console de véhicule automobile, par exemple dans le tableau de bord. Dans les exemples, l'ouverture est située au fond d'un renfoncement de la paroi 7.

Sur la figure 3, la paroi 7 à proximité de l'ouverture 6 présente une forme d'entonnoir au fond duquel se trouve l'ouverture 6. La paroi 7 présente, à proximité de l'ouverture, une surface continue, sans discontinuité, tel que visible sur la figure 4. Le bord inférieur 2 du diffuseur 1 est alors enfoncé dans cette forme d'entonnoir jusqu'à l'ouverture 6. La protubérance 5 est réalisée avec des dimensions supérieures au fond de la forme d'entonnoir (et donc de l'ouverture 6) de sorte qu'elle exerce une pression suffisante contre le pourtour 8 de l'ouverture 6 pour maintenir le diffuseur en place.

Le matériau utilisé et/ou la forme du bord inférieur, sont de préférence choisis afin de permettre une légère déformation de la protubérance et/ou du bord suffisante pour assurer une pression sur la paroi 7 suffisamment élevée pour maintenir le diffuseur autour de l'ouverture 6. La protubérance 5, s'étendant sur toute la périphérie de l'ouverture 6, exerce également une fonction d'étanchéité à l'air limitant, voire empêchant, le passage de celui-ci entre la paroi 7 et la paroi latérale 3 du diffuseur, de sorte que le flux d'air ne peut décoller le diffuseur de la paroi 7.

Dans l'exemple, la paroi latérale 3 du diffuseur présente une forme sensiblement similaire à la paroi 7 de l'aérateur sur lequel il doit être placé.

Sur la figure 5, la paroi 7 présente à proximité de l'ouverture 6 de l'aérateur un saut de section augmentant le diamètre de la forme d'entonnoir à proximité de l'ouverture 6. Un épaulement 9 est ainsi formé qui va pouvoir servir d'appui à la protubérance 5 du diffuseur, tel que visible sur la figure 6. De même que dans l'exemple précédent, le diamètre externe de la protubérance 5 est, de préférence, légèrement supérieur au diamètre élargi de la paroi 7 à proximité de l'ouverture 6, afin d'éviter le passage d'un flux d'air entre les parois 7 et 3, et d'éviter ainsi le décollement du diffuseur.

Le diffuseur selon l'invention a été décrit pour une ouverture de forme circulaire. Il va de soi qu'il peut également être utilisé avec une ouverture de forme différente, ovale, rectangulaire ou autre.

La forme générale du diffuseur selon l'invention va ainsi varier en fonction de l'aérateur auquel il est destiné.

De même, la paroi latérale 3 du diffuseur est conformée en fonction de la forme de la paroi 7 proche de l'ouverture de l'aérateur auquel le diffuseur est destiné. Elle peut donc présenter des formes très variées. Elle peut toutefois également présenter une forme évasée différente de la forme de la paroi de l'aérateur 7.

Dans l'exemple, la protubérance 5, située au niveau du bord inférieur 2, sert à la fois de moyens de maintien et d'étanchéité du diffuseur. Toutefois, on peut envisager un diffuseur pourvu uniquement de moyens de maintien (ou dont les moyens de maintien sont distincts des moyens d'étanchéité) solidaires du bord 2. Il peut s'agir par exemple d'une pluralité de pattes aptes à venir en prise avec les ailettes de l'aérateur, ou d'une pluralité de protubérances distinctes réparties sur la périphérie du bord 2, d'une manière similaire à la protubérance 5 décrite.

Dans l'exemple, le diffuseur est réalisé d'une seule matière, en une seule pièce, on pourrait toutefois envisager que les moyens de maintien et/ou d'étanchéité soient réalisés en une autre matière que celle formant la paroi latérales et solidarisés au bord inférieur pendant ou après la fabrication de ce dernier.

## Revendications

1. Diffuseur de substance(s) volatile(s) (1) destiné à un aérateur de véhicule automobile du type présentant une ouverture (6) située au fond d'un renfoncement, avec un bord inférieur (2) sensiblement de même forme et de mêmes dimensions que le pourtour (8) de l'ouverture (6) dudit aérateur, **caractérisé en ce qu'**il présente une paroi latérale (3), en un matériau apte à être imprégné d'une ou plusieurs substances volatiles, s'étendant depuis le bord (2) en s'évasant, et **en ce que** le bord (2) est pourvu de moyens de maintien (5) aptes à maintenir le diffuseur sur le pourtour de l'ouverture de l'aérateur

2. Diffuseur selon la revendication 1, **caractérisé en ce qu'**il est pourvu de moyens d'étanchéité (5) aptes à limiter le passage d'un flux d'air entre le bord (2) du diffuseur et le pourtour (8) de l'ouverture de l'aérateur.

3. Diffuseur selon la revendication 1 ou 2, **caractérisé en ce que** les moyens de maintien et/ou d'étanchéité sont formés d'une protubérance (5) s'étendant sur la face externe du bord (2) sur toute la périphérie de celui-ci, les dimensions les plus grandes de la protubérance (5) étant légèrement supérieures aux dimensions du pourtour (8) de l'ouverture (6) de l'aérateur.

4. Diffuseur selon l'une des revendications 1 à 3, **caractérisé en ce que** la paroi latérale (3) présente sensiblement la même forme que la paroi (7) entourant l'ouverture (6) de l'aérateur.

5. Diffuseur selon l'une des revendications 1 à 4, **caractérisé en ce que** la paroi latérale (3) est formée d'une pièce avec le bord inférieur (2).

6. Diffuseur selon l'une des revendications 1 à 5, **caractérisé en ce qu'**il est en matériau recyclable.

7. Diffuseur selon l'une des revendications 1 à 6, **caractérisé en ce qu'**il est en matériau apte à être moulé.

8. Diffuseur selon l'une des revendications 1 à 7, **caractérisé en ce qu'**il est en papier ou carton.

## Claims

1. Volatile substance(s) dispenser (1) for a motor vehicle air vent of the type having an opening (6) at the bottom of a recess, with a lower edge (2) having essentially the same shape and dimensions as the periphery (8) of the opening (6) of said air vent, **characterized in that** it has a side wall (3.) which is made of a material that can be impregnated with one or more volatile substances and which flares out from the edge (2), and **in that** the edge (2) has holding means (5) for holding the dispenser on the periphery of the air vent opening.

2. Dispenser according to Claim 1, **characterized in that** it has sealing means (5) that can limit the passage of an air flow between the edge (2) of the dispenser and the periphery (8) of the air vent opening.

3. Dispenser according to Claim 1 or 2, **characterized in that** the holding and/or sealing means consist of a protuberance (5) extending around the external face of the edge (2) over the whole of its periphery, the greatest dimensions of the protuberance (5) being slightly greater than the dimensions of the periphery (8) of the air vent opening (6).

4. Dispenser according to one of Claims 1 to 3, **characterized in that** the side wall (3) has essentially the same shape as the wall (7) surrounding the opening (6) of the air vent.

5. Dispenser according to one of Claims 1 to 4, **characterized in that** the side wall (3) is made as a single piece with the lower edge (2).

6. Dispenser according to one of Claims 1 to 5, **characterized in that** it is made of a recyclable material.

7. Dispenser according to one of Claims 1 to 6, **characterized in that** it is made of a mouldable material.

8. Dispenser according to one of Claims 1 to 7, **characterized in that** it is made of paper or cardboard.

## Patentansprüche

1. Zerstäuber für flüchtige Substanz(en) (1), der für einen Kraftfahrzeugbelüfter des Typs vorgesehen ist, der eine Öffnung (6) aufweist, die sich im Boden eines Rücksprungs befindet, wobei ein unterer Rand (2) im Wesentlichen die gleiche Form und die gleichen Abmessungen wie der Umfang (8) der Öffnung (6) des Belüfters hat, **dadurch gekennzeichnet, dass** er eine Seitenwand (3) aufweist, die aus einem mit einer oder mit mehreren flüchtigen Substanzen imprägnierten Material besteht und die sich von dem Rand (2) erstreckt und sich dabei erweitert, und dass der Rand (2) mit Haltemitteln (5) versehen ist, die den Zerstäuber am Umfang der Öffnung des Belüfters halten können.

2. Zerstäuber nach Anspruch 1, **dadurch gekennzeichnet, dass** er mit Dichtungsmitteln (5) versehen ist, die den Durchgang eines Luftstroms zwischen dem Rand (2) des Zerstäubers und dem Umfang (8) der Öffnung des Belüfters begrenzen können.

3. Zerstäuber nach Anspruch 1 oder 2, **dadurch gekennzeichnet, dass** die Halte- und/oder Dichtungsmittel aus einem Vorsprung (5) gebildet sind, der sich an der Außenseite des Randes (2) um dessen gesamten Umfang erstreckt, wobei die größten Abmessungen des Vorsprungs (5) etwas größer als die Abmessungen des Umfangs (8) der Öffnung (6) des Belüfters sind.

4. Zerstäuber nach einem der Ansprüche 1 bis 3, **dadurch gekennzeichnet, dass** die Seitenwand (3) im Wesentlichen die gleiche Form wie die die Öffnung (6) des Belüfters umgebende Wand (7) hat.

5. Zerstäuber nach einem der Ansprüche 1 bis 4, **dadurch gekennzeichnet, dass** die Seitenwand (3) einteilig mit dem unteren Rand (2) ausgebildet ist.

6. Zerstäuber nach einem der Ansprüche 1 bis 5, **dadurch gekennzeichnet, dass** er aus einem recyclingfähigen Material besteht.

7. Zerstäuber nach einem der Ansprüche 1 bis 6, **dadurch gekennzeichnet, dass** er aus einem gießfähigen Material besteht.

8. Zerstäuber nach einem der Ansprüche 1 bis 7, **dadurch gekennzeichnet, dass** er aus Papier oder Karton besteht.
